# EUROPEAN PATENT APPLICATION

(11) **EP 4 471 134 A1**
(43) Date of publication of application: **04.12.2024**
(21) Application number: 22922955.4
(22) Date of filing: 30.01.2022
(51) Int. Cl.: C12N 9/00, C12Q 1/6844

(54) **METHOD FOR DETECTING TARGET NUCLEIC ACID ON THE BASIS OF PRIMER-AND-ENZYME INTEGRATED PELLET**

(71) Applicant: Oxford University (Suzhou) Science & Technology Co., Ltd., Suzhou, Jiangsu 215123 (CN)
(72) Inventor: YEUNG, Danny Sheng Wu, Hong Kong 999077 (CN); MA, Wu-Po, Hong Kong 999077 (CN); HSU, Chia-Chen, Hong Kong 999077 (CN); TSAI, Ming-Shan, Hong Kong 999077 (CN)
(74) Representative: v. Bezold & Partner Patentanwälte - PartG mbB
(86) International application number: PCT/CN2022/075267
(87) International publication number: WO 2023/142131

(57) **Abstract**

The present invention provides an enzyme composition and a preparation method therefor and a use thereof. The enzyme composition includes an enzyme, an enzyme stabilizer, a filler, and a primer designed for a target nucleic acid. The enzyme composition can be preserved at room temperature and maintain good stability and activity, thereby improving adaptability of the enzyme composition to an ambient temperature. In addition, the enzyme is integrated with the primer, so that the enzyme composition is used in PCR detection, especially in LAMP nucleic acid amplification, a time for preparing a reaction system is shortened, and an operation procedure is simplified. Therefore, the enzyme composition is suitable for large-scale production. The enzyme composition is applied to target nucleic acid detection to maintain high sensitivity and detection stability.

## Description

### TECHNICAL FIELD

The present invention relates to the field of biochemical reagents. Specifically, the present invention relates to a target nucleic acid detection method based on a primer-enzyme integrated pellet.

### BACKGROUND

A DNA polymerase is a type of enzyme for biologically catalyzing a deoxyribonucleic acid (DNA) and a ribonucleic acid (RNA), is a necessary enzyme complex in a DNA biosynthesis process, and has catalytic activity for DNA synthesis and complementary activity. With development of molecular biology, a polymerase chain reaction (PCR) technology was invented by Mullis of the United States in 1985 and has been rapidly developed. Currently, the polymerase chain reaction technology has become an extremely important technological means in the fields of molecular biology, life sciences, and the like, and is widely applied in the biological fields such as gene amplification, gene cloning, gene modification, analysis of infectious disease sources, and genetic fingerprint identification. As one of important factors of the PCR technology, the DNA polymerase plays an important role in a PCR process. In a sense, the PCR technology is a DNA polymerase technology. Currently, discovery of a thermostable DNA polymerase enables the PCR technology to be much more convenient, and greatly reduces costs of the PCR technology, thereby promoting development and application of the PCR technology. However, currently, to maintain the activity of the DNA polymerase for a long time, preservation and transportation of the DNA polymerase still need to be performed at -20°C, resulting in high costs, inconvenient operation and transportation, and varying degrees of reduction in activity of the enzyme. In recent years, there are many reports on researches of stability and activity of other enzymes during preservation and transportation, but there are few reports on a polymerase. In particular, since the outbreak of SARS-CoV-19, PCR nucleic acid detection of a sample has become a main way to detect a positive or negative sample virus. Therefore, a problem of transporting and preserving the polymerase becomes a problem that needs to be resolved urgently currently.

In 2000, a Japanese scientist Notomi published a new isothermal nucleic acid amplification technology suitable for gene diagnosis in the journal Nucleic Acids Res, that is, loop-mediated isothermal amplification, which is referred to as a "LAMP" technology for short. The "LAMP" technology is to design four or six specific primers for a target gene, and perform isothermal amplification at 60°C to 65°C under the action of a strand displacement DNA polymerase (Bst DNA Polymerase) for 15 minutes to 60 minutes, to implement 10⁹-10¹⁰-fold nucleic acid amplification.

Compared with conventional PCR, the "LAMP" technology has the following advantages:
1. Sensitivity is high, and is two to five orders of magnitude higher than that of a conventional nucleic acid amplification method.
2. A reaction time is short, there is no need to perform thermal denaturation on a template, and a reaction can be completed within 30 minutes to 60 minutes.
3. An entire reaction process is thermostatic without temperature cycling and without relying on an expensive professional detection device in clinical use.
4. A reaction result may be determined by observing a color developer visually, there is no need to perform electrophoresis and ultraviolet observation, the reaction result is determined simply and fast, and an ordinary person who does not have a professional skill can perform quick diagnosis in a disease scene.

In recent years, the "LAMP" technology has been widely applied in many countries to quickly detect pathogenic microorganisms such as SARS, influenza virus, mycoplasma pneumoniae, and mycobacterium tuberculosis. However, in actual application, a polymerase required for a LAMP reaction needs to be preserved and transported at a low temperature below -20°C for a long time, resulting in high costs, inconvenient operation and transportation, and varying degrees of reduction in activity of the enzyme.

In addition, in a process of detecting a target nucleic acid through PCR, the polymerase and a primer are separated in most cases, resulting in an increase in a workload of an operator. Therefore, it is urgent to develop an enzyme composition that can be preserved at room temperature and integrates a primer and an enzyme.

### SUMMARY

The present invention is intended to resolve, at least to some extent, one of technical problems in the related art. Therefore, an objective of the present invention is to provide an enzyme composition. The enzyme composition can be preserved at room temperature and maintain good stability and activity, thereby improving adaptability of the enzyme composition to an ambient temperature. In addition, an enzyme is integrated with a primer, so that the enzyme composition is used in PCR detection, especially in LAMP nucleic acid amplification, a time for preparing a reaction system is shortened, and an operation procedure is simplified. Therefore, the enzyme composition is suitable for large-scale production. The enzyme composition is applied to target nucleic acid detection to maintain high sensitivity and detection stability.

In one aspect of the present invention, the present invention provides an enzyme composition. According to an embodiment of the present invention, the enzyme composition is suitable for preservation at room temperature. The enzyme composition includes:
an enzyme;
an enzyme stabilizer;
a filler; and
a primer designed for a target nucleic acid.

The enzyme stabilizer is selected from optional sucrose and at least one of trehalose or trehalose dihydrate.

The filler is selected from at least one of dextran, mannitol, polyethylene glycol, bovine serum albumin, polyvinyl alcohol, raffinose, or sorbitol.

Since the outbreak of SARS-CoV-2, PCR nucleic acid detection has become a main way to detect a positive or negative sample virus. To maintain activity of a DNA polymerase during preservation and transportation, cold-chain transportation, which means to maintain a preservation or transportation temperature at -20 degrees Celsius, is an existing manner used on a large scale. However, cold-chain transportation increases preservation and transportation costs and is complicated in operation. The inventors find that by adding the primer and the special enzyme stabilizer and filler, for example, a trehalose dihydrate enzyme stabilizer and a dextran filler, to a liquid enzyme preparation, and lyophilizing the liquid enzyme preparation to prepare a lyophilized product, the enzyme can be preserved at room temperature. In addition, the enzyme is integrated with the primer, so that the liquid enzyme preparation is used in LAMP detection, a time for preparing a reaction system is shortened, and an operation procedure is simplified. Activity and stability of the enzyme can be maintained by the lyophilized product that is prepared by integrating the enzyme with the primer. Therefore, the product can withstand environmental instability, can be preserved at room temperature for 1 to 7 days, and is suitable for large-scale production.

According to an embodiment of the present invention, a weight ratio of the enzyme stabilizer to the enzyme is (5-20): 100, and a weight ratio of the filler to the enzyme is (1-15): 100. Therefore, the activity of the enzyme can be maintained and the enzyme composition can be maintained in a stable form at room temperature.

According to an embodiment of the present invention, the enzyme composition is a lyophilized product obtained through lyophilization.

According to an embodiment of the present invention, the lyophilized product is lyophilized powder or a lyophilized pellet.

According to an embodiment of the present invention, the enzyme composition is a pellet, and a diameter of the pellet is 0.1 mm to 20 mm.

According to an embodiment of the present invention, the enzyme in the enzyme composition is selected from at least one of a DNA polymerase, an RNA polymerase, a reverse transcriptase, or a recombinase.

In another aspect of the present invention, the present invention provides a method for preparing the foregoing enzyme composition. According to an embodiment of the present invention, the method includes:
(i) mixing an enzyme-containing preparation, the enzyme stabilizer, the filler, and the primer designed for a target nucleic acid; and
(ii) lyophilizing a mixture obtained in step (i) to obtain a lyophilized product A;
or
(I) mixing an enzyme-containing preparation, an enzyme stabilizer, and a filler to obtain a first mixture; and
(II) mixing a primer designed for a target nucleic acid with at least a part of the first mixture obtained in step (I) to obtain a second mixture, and then performing lyophilization to obtain a lyophilized product B;
or
(a) mixing an enzyme-containing preparation, an enzyme stabilizer, and a filler to obtain a first mixture;
(b) lyophilizing the first mixture obtained in step (a) to obtain a lyophilized product C; and
(c) mixing a primer designed for a target nucleic acid with an additional enzyme stabilizer, performing lyophilization to obtain a lyophilized product D, and mixing the lyophilized products C and D to obtain the enzyme composition.

According to an embodiment of the present invention, an enzyme in the enzyme-containing preparation is a DNA polymerase or a mixture of a DNA polymerase and a reverse transcriptase.

According to an embodiment of the present invention, the enzyme stabilizer is trehalose dihydrate.

According to an embodiment of the present invention, the filler is dextran.

According to an embodiment of the present invention, a concentration of the enzyme is 0.1 U/µL to 5.0 U/µL.

According to an embodiment of the present invention, a weight ratio of the enzyme stabilizer to the enzyme is (5-20): 100.

According to an embodiment of the present invention, a weight ratio of the filler to the enzyme is (1-15): 100.

According to an embodiment of the present invention, a concentration of the enzyme stabilizer is 0.05 mg/µL to 0.2 mg/µL.

According to an embodiment of the present invention, a concentration of the filler is 0.01 mg/µL to 0.15 mg/µL.

According to an embodiment of the present invention, a concentration of the primer designed for the target nucleic acid is 0.1 µM to 6.4 µM.

The target nucleic acid can be stably and accurately detected through target nucleic acid amplification detection with such a primer concentration.

According to an embodiment of the present invention, the lyophilization is implemented by the following steps:
1) contacting the mixture with liquid nitrogen dropwise to form a plurality of pellets; and
2) performing vacuum lyophilization on the plurality of pellets at -35°C to - 80°C to obtain the enzyme composition.

A time for the vacuum lyophilization is at least 35 h.

According to an embodiment of the present invention, a temperature for the vacuum lyophilization is -35°C to -60°C.

According to an embodiment of the present invention, a temperature for the vacuum lyophilization is -40°C to -45°C.

When the method for preparing the enzyme composition, especially for preparing a spherical enzyme (also referred to as "enzyme pellets" or "lyobeads") is used, the primer and the specific enzyme stabilizer and filler are added to a liquid enzyme preparation (the enzyme-containing preparation), liquid nitrogen freezing is performed to form beads, and then vacuum lyophilization is further performed under a specific condition to remove water, to obtain the lyobeads that have a complete shape and good mechanical performance and can be preserved and transported at room temperature. In addition, the enzyme lyobeads have a high volume-surface area ratio, so that rapid resolubilization is implemented.

The pellets obtained after liquid nitrogen freezing still contain a lot of water that hydrolyzes a protein during preservation and transportation. To maintain activity of the protein and maintain stability of the protein at room temperature, the inventors perform vacuum lyophilization on the pellets. Hydrogen-bond interactions between the protein and a water molecule are reduced by removing water, resulting in a change in a three-dimensional structure of the protein and denaturation. Therefore, the specific enzyme stabilizer needs to be added to reduce changes in the structure of the protein in a drying process. Water exists in the pellets in a form of an ice crystal after liquid nitrogen is added dropwise. A hole is left due to sublimation of the ice crystal in the drying process. Therefore, the inventors add the specific filler before adding liquid nitrogen dropwise to improve mechanical strength of the pellets and maintain a porous structure of the pellets, thereby preventing the pellets from being collapsed, preventing water content of a final product from being affected, and preventing a preservation period of the product from being shortened.

According to an embodiment of the present invention, the room temperature is 20°C to 40°C.

In still another aspect of the present invention, the present invention provides a use of the foregoing enzyme composition or the enzyme composition prepared according to the foregoing method in preparing a kit. According to an embodiment of the present invention, the kit is used for detecting a target nucleic acid.

According to an embodiment of the present invention, the enzyme contained in the enzyme composition is selected from at least one of a DNA polymerase, an RNA polymerase, or a reverse transcriptase.

According to an embodiment of the present invention, the nucleic acid is a DNA or an RNA.

In still another aspect of the present invention, the present invention provides a kit. According to an embodiment of the present invention, the kit contains the foregoing enzyme composition or the enzyme composition prepared according to the foregoing method.

According to an embodiment of the present invention, the enzyme contained in the enzyme composition is selected from at least one of a DNA polymerase, an RNA polymerase, or a reverse transcriptase.

In still another aspect of the present invention, the present invention provides a nucleic acid amplification reaction system. According to an embodiment of the present invention, the nucleic acid amplification reaction system includes the foregoing enzyme composition or the enzyme composition prepared according to the foregoing method.

According to an embodiment of the present invention, the nucleic acid amplification reaction system further includes dNTP and a reaction buffer for nucleic acid amplification.

According to an embodiment of the present invention, the nucleic acid amplification reaction system further includes a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

According to an embodiment of the present invention, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

According to an embodiment of the present invention, the fungus is selected from at least one of yeast, mold, candida albicans, cryptococcus, aspergillus, or mucor, and the chlamydia is chlamydia trachomatis.

According to an embodiment of the present invention, the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

According to an embodiment of the present invention, the coronavirus is selected from at least one of SARS-CoV, SARS-CoV-2, SARS-CoV-19, MERS-CoV, HCoV-229E, HCoV-OC43, HCoV-NL63, or HCoV-HKU1.

According to an embodiment of the present invention, the influenza virus is selected from influenza A virus and influenza B virus.

According to an embodiment of the present invention, the protozoan is selected from at least one of plasmodium, trichomonas vaginalis, toxoplasma gondii, leishmania, babesia, entamoeba histolytica, acanthamoeba, or trypanosoma brucei.

In still another aspect of the present invention, the present invention provides a use of the foregoing enzyme composition, the enzyme composition prepared according to the foregoing method, the foregoing kit, or the foregoing nucleic acid amplification reaction system in target nucleic acid detection.

In still another aspect of the present invention, the present invention provides a LAMP (Loop-mediated isothermal amplification) reaction system. According to an embodiment of the present invention, the LAMP reaction system includes the foregoing enzyme composition or the enzyme composition prepared according to the foregoing method.

The foregoing enzyme composition is applied to the LAMP reaction system, especially, a primer and a mixture of a DNA polymerase and a reverse transcriptase are prepared into lyophilized pellets, so that the enzyme does not need to be preserved at a low temperature and can be directly preserved at room temperature to maintain good stability and activity, thereby improving adaptability of the enzyme to an ambient temperature. In addition, enzyme content and primer content of the enzyme pellet may be controlled as required, so that the enzyme pellet is used in a reaction system. When sample addition is performed in a LAMP reaction, only one to more lyophilized pellets need to be added without performing repeated addition by using a pipette, thereby avoiding a problem that the enzyme is easily contaminated and inactivated when an existing liquid enzyme and the primer are added to the reaction system, shortening a time for preparing the LAMP reaction system, and improving working efficiency.

According to an embodiment of the present invention, the enzyme in the enzyme composition is a DNA polymerase or a mixture of a DNA polymerase and a reverse transcriptase.

According to an embodiment of the present invention, the DNA polymerase is a Bst DNA polymerase.

According to an embodiment of the present invention, the LAMP reaction system further includes dNTP and a reaction buffer for nucleic acid amplification.

According to an embodiment of the present invention, the LAMP reaction system further includes a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

According to an embodiment of the present invention, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

According to an embodiment of the present invention, the fungus is selected from at least one of yeast, mold, candida albicans, cryptococcus, aspergillus, or mucor, and the chlamydia is chlamydia trachomatis.

According to an embodiment of the present invention, the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

According to an embodiment of the present invention, the coronavirus is selected from at least one of SARS-CoV, SARS-CoV-2, SARS-CoV-19, MERS-CoV, HCoV-229E, HCoV-OC43, HCoV-NL63, or HCoV-HKU1.

According to an embodiment of the present invention, the influenza virus is selected from influenza A virus and influenza B virus.

According to an embodiment of the present invention, the protozoan is selected from at least one of plasmodium, trichomonas vaginalis, toxoplasma gondii, leishmania, babesia, entamoeba histolytica, acanthamoeba, or trypanosoma brucei.

In still another aspect of the present invention, the present invention provides a method for detecting a target nucleic acid in a to-be-detected sample. According to an embodiment of the present invention, the method includes:
(i). obtaining the to-be-detected sample or obtaining a nucleic acid isolated from the to-be-detected sample;
(ii). using the to-be-detected sample or the nucleic acid as a template, and amplifying the template by using the foregoing enzyme composition or the enzyme composition prepared according to the foregoing method, to obtain an amplified product; and
(iii). detecting whether the amplified product contains a plurality of copies of the target nucleic acid, where if the amplified product contains the plurality of copies of the target nucleic acid, it indicates that the to-be-detected sample contains the target nucleic acid.

According to an embodiment of the present invention, the method further includes:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample.

According to an embodiment of the present invention, the reference sequence is a nucleic acid sequence of a wild-type target nucleic acid that is not mutated.

According to an embodiment of the present invention, the nucleic acid is a DNA or an RNA.

According to an embodiment of the present invention, the to-be-detected sample is derived from a plant, an animal, or an environment sample.

According to an embodiment of the present invention, the animal may be a mammal, and the mammal is selected from a human, a rat, a mouse, a pig, cattle, a sheep, a dog, and a cat.

Additional aspects and advantages of the present invention will be partly given in the following description, and part thereof will become apparent from the following description, or may be learned through the practice of the present invention.

### BRIEF DESCRIPTION OF DRAWINGS

The above-mentioned and/or additional aspects and advantages of the present invention become apparent and easy to understand from the description of the embodiments in conjunction with the following accompanying drawings.
FIG. 1 is a flowchart of a method for preparing an enzyme composition according to an embodiment of the present invention;
FIG. 2 is a flowchart of a method for preparing an integrated enzyme pellet according to an embodiment of the present invention;
FIG. 3 shows a LAMP amplification result of integrated enzyme pellets in a group A according to Embodiment 4;
FIG. 4 shows a LAMP amplification result of integrated enzyme pellets in a group B according to Embodiment 4;
FIG. 5 shows a LAMP amplification result of enzyme pellets in a control group according to Embodiment 4;
FIG. 6 shows a standard curve of 5, 50, 5000, 50000, and 500000 Twist RNAs determined through qPCR according to Embodiment 4;
FIG. 7 shows results obtained after qPCR is repeatedly performed on integrated enzyme pellets in a group A and on a control group for 24 times according to Embodiment 4, where a quantity of SARS-CoV-2 copies on a left side is 48 copies/reaction, and a quantity of SARS-CoV-2 copies on a right side is 96 copies/reaction;
FIG. 8 shows a LAMP amplification result of a mixture of an enzyme pellet and a primer pellet according to Embodiment 6; and
FIG. 9 shows a curve result obtained by measuring a glass transition temperature of integrated enzyme pellets in a group A through differential scanning calorimetry according to Embodiment 7.

### DESCRIPTION OF EMBODIMENTS

The following describes in detail the embodiments of the present invention. The embodiments described below are exemplary and are merely intended to explain the present invention and are not understood as limiting the present invention. If a specific technology or condition is not specified in the embodiments, a technology or condition described in the literatures in the art or a product specification is used. Reagents or instruments used without specified manufacturers are all conventional products that can be obtained from markets.

In the description of the present invention, "a plurality of" means at least two, for example, two or three, unless otherwise specifically limited.

### Enzyme Composition

In an implementation of the present invention, the present invention provides an enzyme composition. The enzyme composition includes an enzyme, an enzyme stabilizer, a filler, and a primer designed for a target nucleic acid. The enzyme stabilizer is selected from optional sucrose and at least one of trehalose or trehalose dihydrate. The filler is selected from at least one of dextran, mannitol, polyethylene glycol, bovine serum albumin, polyvinyl alcohol, raffinose, or sorbitol.

In an implementation of the present invention, a weight ratio of the enzyme stabilizer to the enzyme is (5-20): 100, and a weight ratio of the filler to the enzyme is (1-15):100.

Activity of the enzyme can be maintained and a stable form of the enzyme composition can be maintained at room temperature when the weight ratio of the enzyme stabilizer to the filler falls within this range. With regard to content of the enzyme in the enzyme composition, an amount of enzyme commonly used by a person skilled in the art may be added to a liquid enzyme preparation during preparation of the enzyme composition based on different types of enzymes in the formed enzyme composition and different uses of the enzymes. For example, if the enzyme in the enzyme composition is used for a LAMP reaction, the enzyme contained in the enzyme composition is a mixture of a DNA polymerase and a reverse transcriptase, and a corresponding amount of enzyme is added based on a concentration of a liquid enzyme preparation used for the LAMP reaction.

For example, the enzyme contained in the enzyme composition is a mixture of a Bst DNA polymerase and the reverse transcriptase when the enzyme in the enzyme composition is used for the LAMP reaction, where the weight ratio of the enzyme stabilizer to the enzyme is (5-20): 100, and the weight ratio of the filler to the enzyme is (1-15):100.

Content of the primer designed for the target nucleic acid in the enzyme composition is adjusted based on a concentration of the primer required for a LAMP reaction system. For example, if the primer in the enzyme composition is used for the LAMP reaction, a corresponding amount of primer is added based on a concentration of the primer for the LAMP reaction. Based on this, the enzyme stabilizer and the filler are added, and lyophilization is performed after mixing.

It should be noted that, for the enzyme composition in the present invention, the enzyme, the enzyme stabilizer, the filler, and the primer designed for the target nucleic acid may be integrated into a complete entirety, or the enzyme, a part of the enzyme stabilizer, and a part of the filler form a complete entirety, and the primer, the remaining enzyme stabilizer, and the remaining filler form a complete entirety, where concentrations of the enzyme stabilizer are equal and concentrations of the filler are equal between the two entireties. The complete entirety may exist, for example, in a pellet state.

In an implementation of the present invention, the enzyme composition is a lyophilized product obtained through lyophilization, and the lyophilized product is lyophilized powder or a lyophilized pellet. A form of the lyophilized product is not limited herein. A person skilled in the art may correspondingly adjust the form of the lyophilized product as required by controlling a lyophilization process.

In an implementation of the present invention, the enzyme composition is a pellet, and a diameter of the pellet is 0.1 mm to 20 mm (for example, the diameter may be 0.1 mm, 0.2 mm, 0.3 mm, 0.4 mm, 0.5 mm, 0.6 mm, 0.7 mm, 0.8 mm, 0.9 mm, 1.0 mm, 1.5 mm, 2.0 mm, 2.5 mm, 3.0 mm, 3.5 mm, 4.0 mm, 4.5 mm, 5.0 mm, 5.5 mm, 6.0 mm, 6.5 mm, 7.0 mm, 7.5 mm, 8.0 mm, 8.5 mm, 9.0 mm, 9.5 mm, 10 mm, 11 mm, 12 mm, 13 mm, 14 mm, 15 mm, 16 mm, 17 mm, 18 mm, 19 mm, or 12 mm). When the enzyme exists in the pellet state (also referred to herein as an enzyme pellet or a lyophilized pellet), and the enzyme and the primer need to be added to a reaction system, the enzyme pellet containing the enzyme and the primer can be added directly without using a pipette for a plurality times as adding a liquid enzyme and the primer, thereby shortening a time of an operator and improving efficiency.

In an implementation of the present invention, the enzyme in the enzyme composition is selected from at least one of a DNA polymerase, an RNA polymerase, a reverse transcriptase, or a recombinase. The enzyme in the enzyme composition provided in the present invention is not limited thereto, and the enzyme in the enzyme composition may be an enzyme used in PCR amplification. A person skilled in the art may adjust a type and a dosage of the enzyme and a sequence and a dosage of the primer as required.

### Preparation of an Enzyme Composition

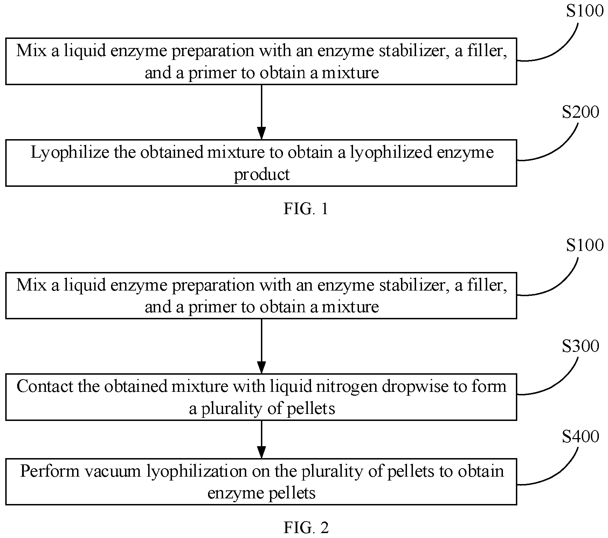

In an implementation of the present invention, the present invention provides a method for preparing the foregoing enzyme composition. As shown in FIG. 1, the method includes the following steps.

S 100: Mix a liquid enzyme preparation with an enzyme stabilizer, a filler, and a primer to obtain a mixture.

S200: Lyophilize the obtained mixture to obtain a lyophilized product.

In an implementation of the present invention, the primer and the liquid enzyme preparation may be mixed into a liquid system, and then the enzyme stabilizer and the filler are added to the liquid system.

In an implementation of the present invention, the method for preparing the foregoing enzyme composition provided in the present invention includes:
(i) mixing an enzyme-containing preparation, the enzyme stabilizer, the filler, and the primer designed for a target nucleic acid; and
(ii) lyophilizing a mixture obtained in step (i) to obtain a lyophilized product A;
or
(I) mixing an enzyme-containing preparation, an enzyme stabilizer, and a filler to obtain a first mixture; and
(II) mixing a primer designed for a target nucleic acid with at least a part of the first mixture obtained in step (I) to obtain a second mixture, and then performing lyophilization to obtain a lyophilized product B;
or
(a) mixing an enzyme-containing preparation, an enzyme stabilizer, and a filler to obtain a first mixture;
(b) lyophilizing the first mixture obtained in step (a) to obtain a lyophilized product C; and
(c) mixing a primer designed for a target nucleic acid with an additional enzyme stabilizer, performing lyophilization to obtain a lyophilized product D, and mixing the lyophilized products C and D to obtain the enzyme composition.

In an implementation of the present invention, an enzyme is a mixture of a DNA polymerase and a reverse transcriptase.

In an implementation of the present invention, the enzyme stabilizer is selected from optional sucrose and at least one of trehalose or trehalose dihydrate.

The enzyme stabilizer may be separate trehalose dihydrate, separate trehalose, a mixture of trehalose dihydrate and sucrose, a mixture of trehalose and sucrose, or a mixture of the three sugars, all of which have a function of stabilizing activity of the enzyme.

In an implementation of the present invention, the enzyme stabilizer is trehalose dihydrate.

In an implementation of the present invention, the filler is selected from at least one of dextran, mannitol, polyethylene glycol, bovine serum albumin, polyvinyl alcohol, raffinose, or sorbitol.

In an implementation of the present invention, the filler is dextran.

In an implementation of the present invention, when the enzyme is a mixture of a DNA polymerase and a reverse transcriptase, a concentration of the enzyme is 0.1 U/µL to 5.0 U/µL. For example, the concentration of the enzyme may be 0.1 U/µL, 0.2 U/µL, 0.3 U/µL, 0.4 U/µL, 0.5 U/µL, 0.6 U/µL, 0.7 U/µL, 0.8 U/µL, 0.9 U/µL, 1.0 U/µL, 1.5 U/µL, 2.0 U/µL, 2.5 U/µL, 3.0 U/µL, 3.5 U/µL, 4.0 U/µL, 4.5 U/µL, or 5.0 U/µL.

In an implementation of the present invention, a weight ratio of the enzyme stabilizer to the enzyme is (5-20): 100.

In an implementation of the present invention, a weight ratio of the filler to the enzyme is (1-15): 100.

In an implementation of the present invention, a concentration of the enzyme stabilizer is 0.05 mg/µL to 0.2 mg/µL. For example, the concentration of the enzyme stabilizer may be 0.05 mg/µL, 0.06 mg/µL, 0.07 mg/µL, 0.08 mg/µL, 0.09 mg/µL, 0.10 mg/µL, 0.11 mg/µL, 0.12 mg/µL, 0.13 mg/µL, 0.14 mg/µL, 0.15 mg/µL, 0.16 mg/µL, 0.17 mg/µL, 0.18 mg/µL, 0.19 mg/µL, or 0.20 mg/µL.

In an implementation of the present invention, a concentration of the filler is 0.01 mg/µL to 0.15 mg/µL. For example, the concentration of the filler may be 0.01 mg/µL, 0.02 mg/µL, 0.03 mg/µL, 0.04 mg/µL, 0.05 mg/µL, 0.06 mg/µL, 0.07 mg/µL, 0.08 mg/µL, 0.09 mg/µL, 0.10 mg/µL, 0.11 mg/µL, 0.12 mg/µL, 0.13 mg/µL, 0.14 mg/µL, or 0.15 mg/µL.

In an implementation of the present invention, a concentration of the primer designed for the target nucleic acid is 0.1 µM to 6.4 µM. For example, the concentration of the primer designed for the target nucleic acid may be 0.1 µM, 0.2 µM, 0.3 µM, 0.4 µM, 0.5 µM, 0.6 µM, 0.7 µM, 0.8 µM, 0.9 µM, 1.0 µM, 1.5 µM, 2.0 µM, 2.5 µM, 3.0 µM, 3.5 µM, 4.0 µM, 4.5 µM, 5.0 µM, 5.5 µM, 6.0 µM, 6.1 µM, 6.2 µM, 6.3 µM, or 6.4 µM.

In another implementation of the present invention, the present invention provides a method for preparing the foregoing enzyme composition. As shown in FIG. 2, the method includes the following steps.

S100: Mix a liquid enzyme preparation with an enzyme stabilizer, a filler, and a primer to obtain a mixture.

S300: Contact the obtained mixture with liquid nitrogen dropwise to form a plurality of pellets.

S400: Perform vacuum lyophilization on the plurality of pellets to obtain enzyme pellets.

In an implementation of the present invention, a temperature for the vacuum lyophilization is -35°C to -80°C, preferably, -35°C to -60°C (for example, -35°C, - 40°C, -45°C, -50°C, -55°C, or -60°C), further preferably, -40°C to -45°C (for example, -40°C, -41°C, -42°C, -43°C, -44°C, or -45°C), and a time for the vacuum lyophilization is at least 35 h.

The obtained mixture of the liquid enzyme preparation, the enzyme stabilizer, the filler, and the primer is in contact with liquid nitrogen dropwise to obtain the plurality of pellets. Because the pellets contain a lot of water in this case, the vacuum lyophilization is performed on the pellets under specific temperature and time conditions to obtain lyophilized enzyme pellets. Because some pores occur in the enzyme pellets after the enzyme pellets loss water, the specific filler is added to ensure integrity and stability of the enzyme pellets at room temperature.

### Use of an Enzyme Composition in Preparing a Kit

In an implementation of the present invention, the foregoing enzyme composition or the enzyme composition prepared according to the foregoing method can be used for preparing a kit, and the kit is used for detecting a target nucleic acid. The foregoing enzyme composition can be used as a part of the kit for detecting the target nucleic acid, and is used in target nucleic acid detection. For example, the foregoing enzyme composition (for example, the enzyme pellets) may be used as a part of a LAMP nucleic acid detection kit to perform a nucleic acid amplification reaction by using the kit, to detect whether a nucleic acid obtained through amplification of a sample is the target nucleic acid.

In an implementation of the present invention, the enzyme contained in the enzyme composition is selected from at least one of a DNA polymerase, an RNA polymerase, or a reverse transcriptase. The enzyme contained in the enzyme composition is not limited thereto. An enzyme composition containing a different enzyme may be prepared according to different requirements of a person skilled in the art. For example, the enzyme contained in the enzyme composition may be a mixture of a Bst DNA polymerase and a reverse transcriptase when the kit is used for a LAMP nucleic acid amplification reaction.

### Kit

In an implementation of the present invention, the present invention provides a kit. The kit contains the foregoing enzyme composition or the enzyme composition prepared according to the foregoing method. The kit may also contain other components according to different detection requirements. For example, if the kit is used for a LAMP nucleic acid amplification reaction, in addition to the enzyme composition, the kit may further contain saline ions (for example, magnesium ions), a buffer, or the like.

### Nucleic Acid Amplification Reaction System

In an implementation of the present invention, the present invention provides a nucleic acid amplification reaction system. The nucleic acid amplification reaction system includes the foregoing enzyme composition or the enzyme composition prepared according to the foregoing method. A nucleic acid may be a DNA or an RNA. The nucleic acid amplification reaction system may further include dNTP and a reaction buffer for nucleic acid amplification.

In an implementation of the present invention, when the nucleic acid amplification reaction system provided in the present invention is used to perform nucleic acid amplification on a to-be-detected sample, the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan. That is, the nucleic acid amplification reaction system may be used to directly perform a LAMP amplification reaction on the bacterial cells or the culture fluid, or perform a LAMP amplification reaction by using the DNA or the RNA as a template.

In an implementation of the present invention, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis. The fungus is selected from at least one of yeast, mold, candida albicans, cryptococcus, aspergillus, or mucor, and the chlamydia is chlamydia trachomatis. The virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus. The coronavirus is selected from at least one of SARS-CoV, SARS-CoV-2, SARS-CoV-19, MERS-CoV, HCoV-229E, HCoV-OC43, HCoV-NL63, or HCoV-HKU1. The influenza virus is selected from influenza A virus and influenza B virus. The protozoan is selected from at least one of plasmodium, trichomonas vaginalis, toxoplasma gondii, leishmania, babesia, entamoeba histolytica, acanthamoeba, or trypanosoma brucei.

### LAMP Reaction System

In an implementation of the present invention, the present invention provides a LAMP reaction system. The LAMP reaction system includes the foregoing enzyme composition or the enzyme composition prepared according to the foregoing method. In an implementation of the present invention, the enzyme in the enzyme composition is a DNA polymerase or a mixture of a DNA polymerase and a reverse transcriptase. The enzyme in the enzyme composition is a mixture of a DNA polymerase and a reverse transcriptase if a nucleic acid contained in a to-be-detected sample is an RNA. The enzyme in the enzyme composition is a DNA polymerase if a nucleic acid contained in a to-be-detected sample is a DNA.

In an implementation of the present invention, the DNA polymerase is a Bst DNA polymerase.

In an implementation of the present invention, the LAMP reaction system further includes dNTP and a reaction buffer for nucleic acid amplification.

In an implementation of the present invention, when the LAMP reaction system provided in the present invention is used to perform nucleic acid amplification on a to-be-detected sample, the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan. That is, the nucleic acid amplification reaction system may be used to directly perform a LAMP amplification reaction on the bacterial cells or the culture fluid, or perform a LAMP amplification reaction by using the DNA or the RNA as a template.

In an implementation of the present invention, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis. The fungus is selected from at least one of yeast, mold, candida albicans, cryptococcus, aspergillus, or mucor, and the chlamydia is chlamydia trachomatis. The virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus. The coronavirus is selected from at least one of SARS-CoV, SARS-CoV-2, SARS-CoV-19, MERS-CoV, HCoV-229E, HCoV-OC43, HCoV-NL63, or HCoV-HKU1. The influenza virus is selected from influenza A virus and influenza B virus. The protozoan is selected from at least one of plasmodium, trichomonas vaginalis, toxoplasma gondii, leishmania, babesia, entamoeba histolytica, acanthamoeba, or trypanosoma brucei.

### Method for Detecting a Target Nucleic Acid in a To-Be-Detected Sample

In an implementation of the present invention, the present invention provides a method for detecting a target nucleic acid in a to-be-detected sample. The method includes the following steps.
(i). Obtain the to-be-detected sample or obtain a nucleic acid isolated from the to-be-detected sample.
(ii). Use the to-be-detected sample or the nucleic acid as a template, and amplify the template by using the foregoing enzyme composition or the enzyme composition prepared according to the foregoing method, to obtain an amplified product.
(iii). Detect whether the amplified product contains a plurality of copies of the target nucleic acid, where if the amplified product contains the plurality of copies of the target nucleic acid, it indicates that the to-be-detected sample contains the target nucleic acid.

In an implementation of the present invention, the method further includes:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample. The reference sequence is a nucleic acid sequence of a wild-type target nucleic acid that is not mutated.

In an implementation of the present invention, the nucleic acid is a DNA or an RNA.

In an implementation of the present invention, the to-be-detected sample is derived from a plant, an animal, a mammal, or an environmental sample.

A source of the to-be-detected sample is not limited thereto, and may alternatively be another species that requires nucleic acid detection.

In an implementation of the present invention, the mammal is selected from a human, a rat, a mouse, a pig, cattle, a sheep, a dog, and a cat. A species of the mammal is not limited thereto, and may alternatively be another animal that requires nucleic acid detection to assess whether the mammal is infected with a pathogenic bacterium or virus.

The following describes the solutions of the present invention with reference to embodiments. A person skilled in the art will understand that the following embodiments are merely intended to describe the present invention, and are not intended to limit the scope of the present invention. If a specific technology or condition is not specified in the embodiments, a technology or condition described in the literatures in the art or a product specification is used. Reagents or instruments used without specified manufacturers are all conventional products that can be obtained from markets.

### Embodiments

### Embodiment 1 Preparation of an Integrated Enzyme Pellet

### Integrated enzyme pellet preparation process

A method for preparing an integrated enzyme pellet (lyophilized pellet) includes the following steps.
(1) Preparation of a mixture of a liquid enzyme preparation, an enzyme stabilizer, a filler, and a primer: performing homogenization on 12.5 µL of WarmStart Colorimetric LAMP2X Master Mix (containing 0.32 U/µL of Bst DNA polymerase and 0.3 U/µL of reverse transcriptase (purchased from NEB, UK, https://intemationaLneb.com/products/ml800-warmstart-colorimetric-lamp-2x-master-mix-dna-rna#Product%20Information)) through transient vortex, adding 1.243 mg of trehalose dihydrate (Sigma-Aldrich, UK) and 1.125 mg of dextran (Sigma-Aldrich, UK) to the WarmStart Colorimetric LAMP2X Master Mix to obtain 13.5 µL of enzyme premix, adding 1 µL of 0.1 µM to 6.4 µM primer (https://sfamjoumals.onlinelibrary.wiley.com/doi/full/10.llll/1751-7915.13586) used for a LAMP amplification reaction for COVID-19 to the enzyme premix, and performing dissolution by inverting a test tube, to obtain a mixture with a total volume of 14.5 µL.
(2) Pellet preparation: loading the mixture obtained in step (1) into a dispenser; dispensing 14.5 µL of reagent to liquid nitrogen; and collecting and transferring a frozen bead to a vial, where the vial contains a thin layer of liquid nitrogen.
(3) Vacuum lyophilization: transferring the bead in step (2) to a lyophilizer (https://www.spscientific.com/Products/Freeze_Dryers_/_Lyophilizers/VirTis/Floor_ Model_Tray/Pilot_Lyophilizers/GenesisPilotLyophilizer/), where the lyophilizer is pre-cooled to -40°C; and dispensing a lyobead to a PCR tube or another IVD device after vacuum drying.

### 2. Vacuum lyophilization

A vacuum lyophilization process is as follows:
A temperature of a drying chamber in the lyophilizer is lower than -40°C before the vial in step (2) is placed in the lyophilizer. The lyophilizer is started according to the following procedure after all vials are loaded (the vials are placed on a shelf).

### 1) Heat treatment phase

**Table 1**

| Heat treatment phase | | | | |
|---|---|---|---|---|
| Step | Shelf temperature (°C) | Time (mins) | Pressure (µBar) | Description |
| The vials are loaded | -50 | N/A | Atmospheric pressure | Hold |
| 1 | -50 to -45 | 10 | 500 | Heat up (Ramp) |
| 2 | -45 | 180 | 500 | Hold |

The following parameters are set for the lyophilizer before primary drying is started.

**Table 2**

| | |
|---|---|
| Freezing temperature | -50°C |
| Additional freezing | 0 min |
| Condenser setting temperature | -60°C |
| Pressure | 500 µBar |

### 2) After relevant parameters are reached, the following drying process starts.

**Table 3**

| Primary drying | | | | |
|---|---|---|---|---|
| Step | Shelf temperature (°C) | Time (mins) | Pressure (µBar) | Description |
| 3 | -45 | 10 | 100 | Hold |
| 4 | -40 | 20 | 100 | Heat up |
| 5 | -40 | 1800 | 100 | Hold |

| Secondary drying | | | | |
|---|---|---|---|---|
| 6 | 20 | 180 | 50 | Heat up |
| 7 | 20 | 400 | 50 | Hold |

Vacuum lyophilization is performed for 40 h. The lyophilizer is backfilled to a pressure of 500 mBar with nitrogen after cycling. It is ensured that all the vials subjected to vacuum lyophilization are correctly covered. Then, the vials are preserved at 2°C to 8°C before use. Integrated enzyme pellets in a group A are obtained according to the foregoing method.

### Embodiment 2 Preparation of an Integrated Enzyme Pellet

A method for preparing an integrated enzyme pellet provided in this embodiment is the same as that in Embodiment 1. A difference lies only in reducing a volume of the WarmStart Colorimetric LAMP 2X Master Mix to obtain 12.5 µL of enzyme premix, and adding 1 µL of primer to the enzyme premix, to finally obtain a mixture with a total volume of 13.5 µL. Integrated enzyme pellets in a group B are obtained through vacuum lyophilization.

### Embodiment 3 Evaluation of a Size of an Integrated Enzyme Pellet

### 1. Size of an integrated enzyme pellet

Sizes of the integrated enzyme pellets obtained in Embodiment 1 and Embodiment 2 are measured, as shown in the following Table 4.

**Table 4**

| Sample No. | Diameters of a control group (mm) | Diameters of the integrated enzyme pellets in the group A (mm) | Diameters of the integrated enzyme pellets in the group B (mm) |
|---|---|---|---|
| 1 | 2.60 | 2.68 | 2.63 |
| 2 | 2.68 | 2.57 | 2.76 |
| 3 | 2.60 | 2.58 | 2.61 |
| 4 | 2.73 | 2.58 | 2.63 |
| 5 | 2.61 | 2.47 | 2.75 |
| 6 | 2.76 | 2.61 | 2.61 |
| 7 | 2.78 | 2.57 | 2.57 |
| 8 | / | 2.58 | 2.65 |
| Average value | 2.68 | 2.58 | 2.65 |
| SD | 0.078 | 0.058 | 0.068 |

The control group refers to enzyme pellets that are prepared according to Embodiment 1 and in which the primer is removed. The foregoing results indicate that there is no obvious difference between the diameters of the integrated enzyme pellets prepared according to the methods in Embodiments 1 and 2 and the diameters of the enzyme pellets in the control group, and the diameters are 2.5 mm to 2.7 mm. In addition, a small difference between the diameters of the samples indicates that the sizes of the enzyme pellets prepared according to the method in the present invention are uniform.

### Embodiment 4 Sensitivity Detection

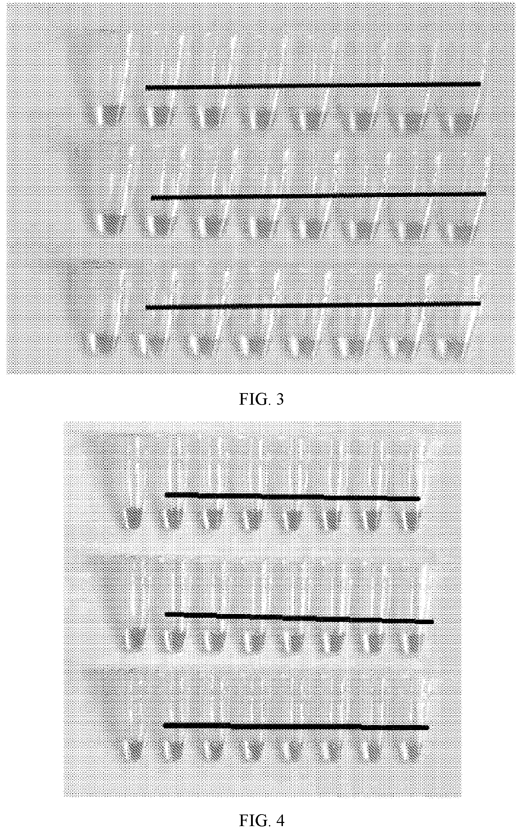

The enzyme pellets in the group A, the group B, and the control group are separately added to a LAMP reaction system (the system contains a phenol red indicator, and a color of phenol red changes with a pH value of a solution in which phenol red is located), and a LAMP amplification reaction (heating for 30 minutes at 65°C) is performed. FIG. 3 to FIG. 5 respectively show amplification results of the group A, the group B, and the control group. These reaction systems are added with different numbers of copies of a Twist SARS-CoV-2 RNA. In an eight-tube strip, 0, 25, 50, 75, 100, 200, 400, and 1000 copies are sequentially added to 25 µL reaction systems respectively from the left to the right.

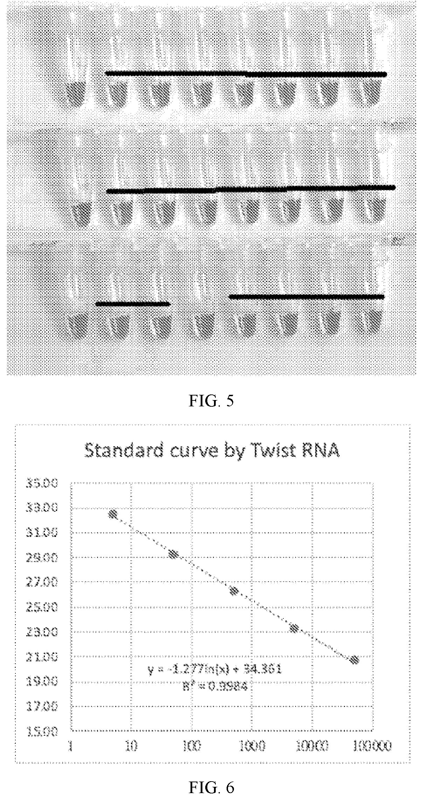

A result of FIG. 3 indicates an LoD performance result of the integrated enzyme pellets in the group A. As low as 25 copies per 25 µL reaction can be detected based on the integrated enzyme pellets in the group A (systems in sample tubes at black horizontal lines are yellow, and systems in the remaining sample tubes are red). A result of FIG. 4 indicates an LoD performance result of the integrated enzyme pellets in the group B. As low as 25 copies per 25 µL reaction can be detected based on the integrated enzyme pellets in the group B (systems in sample tubes at black horizontal lines are yellow, and systems in the remaining sample tubes are red). A result of FIG. 5 indicates an LoD performance result of the enzyme pellets in the control group. As low as 25 copies per 25 µL reaction can be detected based on the enzyme pellets in the control group (systems in sample tubes at black horizontal lines are yellow, and systems in the remaining sample tubes are red). The results of FIG. 3 to FIG. 5 indicate that, when the integrated enzyme pellets containing the enzyme and the primer or the enzyme pellets without the primer that are prepared according to the method in the present invention are applied to a LAMP reaction, high sensitivity can be achieved, and as low as 25 copies per 25 µL reaction can be detected. In addition, it also indicates that addition of the primer in the integrated enzyme pellets does not affect activity and sensitivity of the enzyme pellets.

A qPCR result of 25 copies theoretically contained in each reaction system indicates that an average Ct value is 29.41 (n=3, SE=0.17). A standard curve (FIG. 6) constructed by using the Twist SARS-CoV-2 RNA proves that a true copy number is 48.4 copies per reaction.

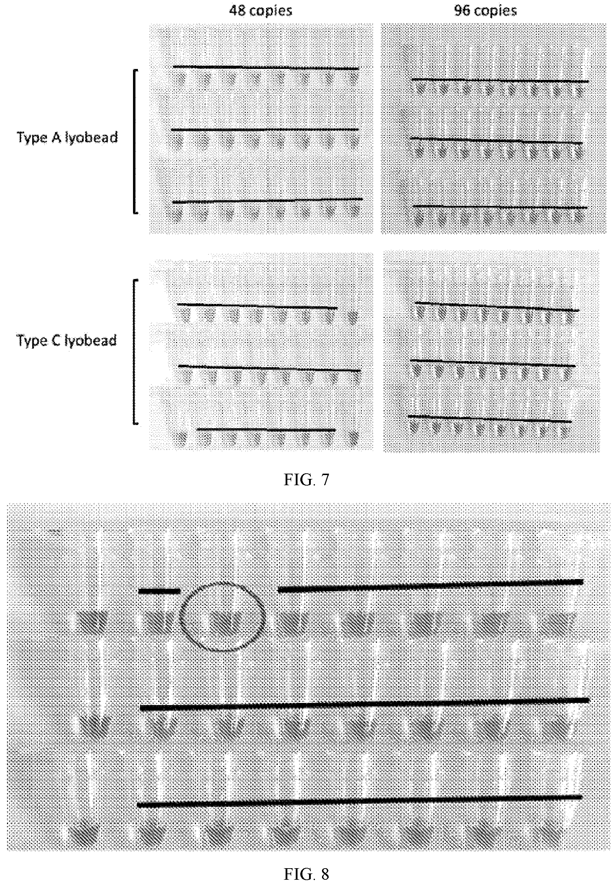

The integrated enzyme pellets in the group A and the group B provided in the present invention have high sensitivity, 48 copies per reaction. A result adjusted based on the qPCR standard curve indicates that an Oxsed RaVid COVID-19 test shows that the enzyme pellets can be used to detect 1.92 copies per µL of SARS-CoV-2 virus particles, that is, 48 copies per 25 µL reaction system can be detected. Results in FIG. 7 indicate that 24 replicates of each of a 48 copies/reaction sample and a 96 copies/reaction sample are virus-positive (all sample tubes are yellow) when the integrated enzyme pellets in the group A are used. However, three of 24 replicates of a 48 copies/reaction sample are still negative (systems in sample tubes at black horizontal lines are red, and systems in the remaining sample tubes are yellow) when the enzyme pellets in the control group are used. Therefore, an LoD of the integrated enzyme pellets in the group A is determined when 48 copies/reaction in all the 24 samples present positive after the reactions are completed. An LoD of the enzyme pellets in the control group is determined when positive 96 copies/reaction in all the 24 samples present positive after the reactions are completed.

### Embodiment 5 Enzyme Pellet and Primer Pellet

### 1. Preparation process of an enzyme pellet and a primer pellet

A method for preparing an enzyme pellet mixture and a primer pellet mixture, includes the following steps.
(1) Preparation of an enzyme premix: performing homogenization on 12.5 µL of WarmStart Colorimetric LAMP 2X Master Mix (containing 0.32 U/µL of Bst DNA polymerase and 0.3 U/µL of reverse transcriptase (purchased from NEB, UK, https://intemational.neb.com/products/m1800-warmstart-colorimetric-lamp-2x-master-mix-dna-rna#Product%20Infonnation)) through transient vortex, and adding 1.243 mg of trehalose dihydrate (Sigma-Aldrich, UK) and 1.125 mg of dextran (Sigma-Aldrich, UK) to the WarmStart Colorimetric LAMP 2X Master Mix, to obtain 13.5 µL of enzyme premix.
(2) Preparation of a primer premix: adding 0.2 µL of 0.22 g/mL trehalose dihydrate (Sigma-Aldrich, UK) to 2.5 µL of 0.1 µM to 6.4 µM primer (https://sfamjoumals.onlinelibrary.wiley.com/doi/full/10.1111/1751-7915.13586) used for a LAMP amplification reaction for COVID-19, to obtain 2.7 µL of primer premix.
(3) Preparation of an enzyme pellet and a primer pellet: respectively loading the mixtures obtained in step (1) and step (2) into dispensers; dispensing reagents to liquid nitrogen; and collecting and transferring a frozen bead to a vial, where the vial contains a thin layer of liquid nitrogen.
(4) Vacuum lyophilization: transferring the bead in step (3) to a lyophilizer, where the lyophilizer is pre-cooled to -40°C; and dispensing a lyobead to a PCR tube or another IVD device after vacuum drying.

A vacuum lyophilization process is the same as that in Embodiment 1. Therefore, independent enzyme pellet and primer pellet (primer lyobead) are obtained.

### Embodiment 6 Sensitivity Detection

Sensitivity of the the enzyme pellet mixture and the primer pellet mixture obtained in Embodiment 5 is detected by using the same method as in Embodiment 4. Reaction systems are added with different numbers of copies of a Twist SARS-CoV-2 RNA. In an eight-tube strip, 0, 25, 50, 75, 100, 200, 400, and 1000 copies are sequentially added to 25 µL reaction systems respectively from the left to the right. A result of FIG. 8 indicates an LoD performance result of the enzyme pellet mixture and the primer pellet mixture. A first column is not added with a template and is red, a sample in a circle is also red, and the remaining samples are all yellow. It indicates that as low as 25 copies per 25 µL reaction can be detected by using the enzyme pellet mixture and the primer pellet mixture.

### Embodiment 7 Stability Evaluation-Measurement of a Glass Transition Temperature of an Integrated Enzyme pellet

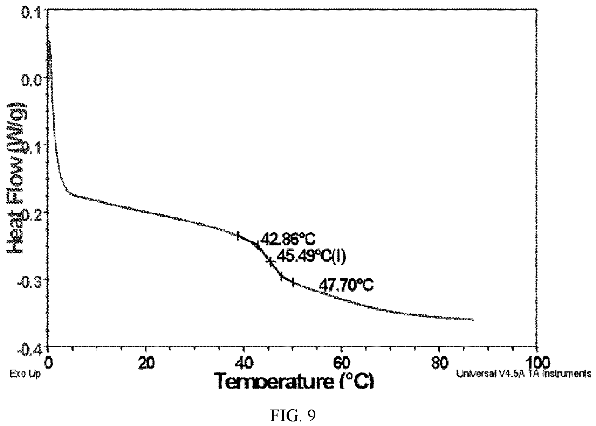

A pellet glass transition temperature is measured by using the integrated enzyme pellets in the group A in Embodiment 1 through differential scanning calorimetry (DSC). Measurement is performed immediately after the pellets are taken out. After thermal equilibrium is performed on a sample in a machine at 0 degrees Celsius for 2 minutes, the machine begins to heat up to 90 degrees Celsius at a rate of 10 degrees Celsius per minute. A heat flow during a heating process is recorded. Because a thermal capacity of a product changes during glass transition, there is a heat absorption process. Therefore, the glass transition temperature of the pellets is measured to be 45.49°C (FIG. 9), that is, the pellets can be relatively stable when being preserved at a temperature below 45 degrees Celsius or even at a lower temperature.

In the description of this specification, reference terms such as "an embodiment", "some embodiments", "example", "specific example", or "some examples" mean that specific features, structures, materials, or characteristics described with reference to the embodiments or examples are included in at least one embodiment or example of the present invention. In this specification, the example expressions of the previous terms are not necessarily with respect to the same embodiments or examples. In addition, the described specific features, structures, materials, or characteristics can be combined in a proper manner in any one or more of the embodiments or examples. In addition, a person skilled in the art can integrate and combine different embodiments or examples and features in different embodiments or examples described in this specification, provided that they do not conflict with each other.

Although the embodiments of the present invention have been shown and described above, it can be understood that the above-mentioned embodiments are exemplary and cannot be understood as limiting the present invention, and a person of ordinary skill in the art can make changes, modifications, replacements, and variations to the above-mentioned embodiments within the scope of the present invention.

## Claims

1. An enzyme composition, wherein the enzyme composition is suitable for preservation at room temperature, and the enzyme composition comprises:
an enzyme;
an enzyme stabilizer;
a filler; and
a primer designed for a target nucleic acid;
the enzyme stabilizer is selected from optional sucrose and at least one of trehalose or trehalose dihydrate; and
the filler is selected from at least one of dextran, mannitol, polyethylene glycol, bovine serum albumin, polyvinyl alcohol, raffinose, or sorbitol.

2. The enzyme composition according to claim 1, wherein a weight ratio of the enzyme stabilizer to the enzyme is (5-20): 100, and a weight ratio of the filler to the enzyme is (1-15): 100.

3. The enzyme composition according to claim 1 or 2, wherein the enzyme composition is a lyophilized product obtained through lyophilization.

4. The enzyme composition according to claim 3, wherein the lyophilized product is lyophilized powder or a lyophilized pellet.

5. The enzyme composition according to any one of claims 1 to 4, wherein the enzyme composition is a pellet, and a diameter of the pellet is 0.1 mm to 20 mm.

6. The enzyme composition according to any one of claims 1 to 5, wherein the enzyme in the enzyme composition is selected from at least one of a DNA polymerase, an RNA polymerase, a reverse transcriptase, or a recombinase.

7. A method for preparing the enzyme composition according to any one of claims 1 to 6, wherein the method comprises:
(i) mixing an enzyme-containing preparation, an enzyme stabilizer, a filler, and a primer designed for a target nucleic acid; and
(ii) lyophilizing a mixture obtained in step (i) to obtain a lyophilized product A;
or
(I) mixing an enzyme-containing preparation, an enzyme stabilizer, and a filler to obtain a first mixture; and
(II) mixing a primer designed for a target nucleic acid with at least a part of the first mixture obtained in step (I) to obtain a second mixture, and then performing lyophilization to obtain a lyophilized product B;
or
(a) mixing an enzyme-containing preparation, an enzyme stabilizer, and a filler to obtain a first mixture;
(b) lyophilizing the first mixture obtained in step (a) to obtain a lyophilized product C; and
(c) mixing a primer designed for a target nucleic acid with an additional enzyme stabilizer, performing lyophilization to obtain a lyophilized product D, and mixing the lyophilized products C and D to obtain the enzyme composition.

8. The method according to claim 7, wherein the enzyme in the enzyme-containing preparation is a DNA polymerase or a mixture of a DNA polymerase and a reverse transcriptase.

9. The method according to claim 7 or 8, wherein the enzyme stabilizer is trehalose dihydrate.

10. The method according to any one of claims 7 to 9, wherein the filler is dextran.

11. The method according to any one of claims 8 to 10, wherein a concentration of the enzyme is 0.1 U/µL to 5.0 U/µL.

12. The method according to any one of claims 8 to 11, wherein a weight ratio of the enzyme stabilizer to the enzyme is (5-20): 100.

13. The method according to any one of claims 8 to 12, wherein a weight ratio of the filler to the enzyme is (1-15): 100.

14. The method according to any one of claims 7 to 13, wherein a concentration of the primer designed for the target nucleic acid is 0.1 µM to 6.4 µM.

15. The method according to any one of claims 7 to 14, wherein the lyophilization is implemented by the following steps:
1) contacting the mixture with liquid nitrogen dropwise to form a plurality of pellets; and
2) performing vacuum lyophilization on the plurality of pellets at -35°C to -80°C to obtain the enzyme composition; and
a time for the vacuum lyophilization is at least 35 h.

16. The method according to claim 15, wherein a temperature for the vacuum lyophilization is -35°C to -60°C.

17. The method according to claim 15, wherein a temperature for the vacuum lyophilization is -40°C to -45°C.

18. The method according to any one of claims 7 to 17, wherein the room temperature is 20°C to 40°C.

19. A use of the enzyme composition according to any one of claims 1 to 6 or the enzyme composition prepared according to the method according to any one of claims 7 to 18 in preparing a kit, wherein the kit is used for detecting a target nucleic acid.

20. The use according to claim 19, wherein the enzyme contained in the enzyme composition is selected from at least one of a DNA polymerase, an RNA polymerase, or a reverse transcriptase.

21. The use according to claim 19 or 20, wherein a nucleic acid is a DNA or an RNA.

22. A kit, wherein the kit contains the enzyme composition according to any one of claims 1 to 6 or the enzyme composition prepared according to the method according to any one of claims 7 to 18.

23. The kit according to claim 22, wherein the enzyme contained in the enzyme composition is selected from at least one of a DNA polymerase, an RNA polymerase, or a reverse transcriptase.

24. A nucleic acid amplification reaction system, wherein the nucleic acid amplification reaction system comprises the enzyme composition according to any one of claims 1 to 6 or the enzyme composition prepared according to the method according to any one of claims 7 to 18.

25. The nucleic acid amplification reaction system according to claim 24, wherein the nucleic acid amplification reaction system further comprises dNTP and a reaction buffer for nucleic acid amplification.

26. The nucleic acid amplification reaction system according to claim 24 or 25, wherein the nucleic acid amplification reaction system further comprises a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

27. The nucleic acid amplification reaction system according to claim 26, wherein the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

28. The nucleic acid amplification reaction system according to claim 26, wherein the fungus is selected from at least one of yeast, mold, candida albicans, cryptococcus, aspergillus, or mucor, and the chlamydia is chlamydia trachomatis.

29. The nucleic acid amplification reaction system according to claim 26, wherein the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

30. The nucleic acid amplification reaction system according to claim 29, wherein the coronavirus is selected from at least one of SARS-CoV, SARS-CoV-2, SARS-CoV-19, MERS-CoV, HCoV-229E, HCoV-OC43, HCoV-NL63, or HCoV-HKU1.

31. The nucleic acid amplification reaction system according to claim 29, wherein the influenza virus is selected from influenza A virus and influenza B virus.

32. The nucleic acid amplification reaction system according to claim 26, wherein the protozoan is selected from at least one of plasmodium, trichomonas vaginalis, toxoplasma gondii, leishmania, babesia, entamoeba histolytica, acanthamoeba, or trypanosoma brucei.

33. A use of the enzyme composition according to any one of claims 1 to 6, the enzyme composition prepared according to the method according to any one of claims 7 to 18, the kit according to claim 22 or 23, or the nucleic acid amplification reaction system according to any one of claims 24 to 32 in target nucleic acid detection.

34. A LAMP reaction system, wherein the LAMP reaction system comprises the enzyme composition according to any one of claims 1 to 6 or the enzyme composition prepared according to the method according to any one of claims 7 to 18.

35. The LAMP reaction system according to claim 34, wherein the enzyme in the enzyme composition is a DNA polymerase or a mixture of a DNA polymerase and a reverse transcriptase.

36. The LAMP reaction system according to claim 35, wherein the DNA polymerase is a Bst DNA polymerase.

37. The LAMP reaction system according to any one of claims 34 to 36, wherein the LAMP reaction system further comprises dNTP and a reaction buffer for nucleic acid amplification.

38. The LAMP reaction system according to any one of claims 34 to 37, wherein the LAMP reaction system further comprises a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

39. The LAMP reaction system according to claim 38, wherein the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

40. The LAMP reaction system according to claim 38, wherein the fungus is selected from at least one of yeast, mold, candida albicans, cryptococcus, aspergillus, or mucor, and the chlamydia is chlamydia trachomatis.

41. The LAMP reaction system according to claim 38, wherein the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

42. The LAMP reaction system according to claim 41, wherein the coronavirus is selected from at least one of SARS-CoV, SARS-CoV-2, SARS-CoV-19, MERS-CoV, HCoV-229E, HCoV-OC43, HCoV-NL63, or HCoV-HKU1.

43. The LAMP reaction system according to claim 41, wherein the influenza virus is selected from influenza A virus and influenza B virus.

44. The LAMP reaction system according to claim 38, wherein the protozoan is selected from at least one of plasmodium, trichomonas vaginalis, toxoplasma gondii, leishmania, babesia, entamoeba histolytica, acanthamoeba, or trypanosoma brucei.

45. A method for detecting a target nucleic acid in a to-be-detected sample, wherein the method comprises:
(i) obtaining the to-be-detected sample or obtaining a nucleic acid isolated from the to-be-detected sample;
(ii) using the to-be-detected sample or the nucleic acid as a template, and amplifying the template by using the enzyme composition according to any one of claims 1 to 6 or the enzyme composition prepared according to the method according to any one of claims 7 to 18, to obtain an amplified product; and
(iii) detecting whether the amplified product contains a plurality of copies of the target nucleic acid, wherein if the amplified product contains the plurality of copies of the target nucleic acid, it indicates that the to-be-detected sample contains the target nucleic acid.

46. The method according to claim 45, wherein the method further comprises:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample.

47. The method according to claim 46, wherein the reference sequence is a nucleic acid sequence of a wild-type target nucleic acid that is not mutated.

48. The method according to any one of claims 45 to 47, wherein the nucleic acid is a DNA or an RNA.

49. The method according to any one of claims 45 to 48, wherein the to-be-detected sample is derived from a plant, an animal, or an environment sample.

50. The method according to claim 49, wherein the animal is a mammal, and the mammal is selected from a human, a rat, a mouse, a pig, cattle, a sheep, a dog, and a cat.
